(19) Europäisches Patentamt

European Patent Office

Office européen des brevets

(11) Numéro de publication : **0 507 656 A1**

(12) **DEMANDE DE BREVET EUROPEEN**

(21) Numéro de dépôt : 92400844.4

(22) Date de dépôt : 26.03.92

(51) Int. Cl.⁵ : **B01J 29/04,** B01J 29/32, C07C 2/00

(30) Priorité : 04.04.91 FR 9104224

(43) Date de publication de la demande : 07.10.92 Bulletin 92/41

(84) Etats contractants désignés : DE GB IT NL

(71) Demandeur : INSTITUT FRANCAIS DU PETROLE 4, avenue de Bois-Préau F-92502 Rueil-Malmaison (FR)

(72) Inventeur : **Marcilly, Christian 91 ter rue Condorcet F-78800 Houilles (FR)** Inventeur : **Alario, Fabio 70 bis, rue Georges Clémenceau F-94210 La Varenne (FR)** Inventeur : **Joly, Jean-François 20, rue Béranger F-75003 Paris (FR)** Inventeur : **Le Peltier, Fabienne 6, rue du 4 Septembre F-92500 Rueil Malmaison (FR)**

(54) Catalyseur de type galloaluminosilicate contenant du gallium, un métal noble de la famille du platine et au moins un métal additionnel, et son utilisation en aromatisation des hydrocarbures.

(57) Catalyseur composite qui contient :

une zéolithe de structure MFI sous forme hydrogène, contenant dans sa charpente au moins les éléments silicium, aluminium et/ou gallium ; une matrice ; du gallium ; au moins un métal noble de la famille du platine, au moins un métal additionnel choisi dans le groupe constitué par l'étain, le germanium, l'indium, le cuivre, le fer, le molybdène, le gallium, le thallium, l'or, l'argent, le ruthénium, le chrome, le tungstène et le plomb et éventuellement un composé choisi dans le groupe constitué par les métaux alcalins et les alcalino-terreux,

sa préparation et son utilisation dans les réactions d'aromatisation des hydrocarbures comportant de 2 à 9 atomes de carbone par molécule.

EP 0 507 656 A1

Jouve, 18, rue Saint-Denis, 75001 PARIS

La présente invention concerne un catalyseur, dit catalyseur composite, qui contient :

– une zéolithe de structure MFI sous forme hydrogène, contenant dans sa charpente le silicium et au moins un élément choisi dans le groupe formé par l'aluminium et le gallium,

– une matrice,

– du gallium,

– au moins un métal noble de la famille du platine, au moins un métal additionnel choisi dans le groupe constitué par l'étain, le germanium, l'indium, le plomb, le gallium et le thallium, les métaux du groupe VIII tels que le cuivre, l'or, l'argent, le nickel, le ruthénium, le fer et les métaux du groupe VI tels que le chrome, le molybdène, le tungstène et éventuellement au moins un composé choisi dans le groupe constitué par les métaux alcalins et les alcalino-terreux, lesdits métaux à l'exception du gallium étant désignés par la suite par le terme "métaux", et

sa préparation et son utilisation dans les réactions d'aromatisation des hydrocarbures comportant de 2 à 9 atomes de carbone par molécule.

Les catalyseurs à base de zéolithes dopées au gallium ou au zinc ou au platine sont connus pour être actifs et sélectifs en aromatisation du propane et du butane. Classiquement, les hydrocarbures à plus de 6 atomes de carbone par molécule sont transformés en aromatiques par réformage catalytique en utilisant des catalyseurs du type alumine acide dopée au platine, métal auquel on peut ajouter par exemple de l'étain ou du rhénium. Ces catalyseurs de réformage sont néanmoins très peu performants pour l'aromatisation des hydrocarbures contenant moins de 6 atomes de carbone par molécule. Il y a donc un grand intérêt pratique à trouver des catalyseurs performants pour l'aromatisation des coupes riches en hydrocarbures du type $C_2$-$C_9$.

La réaction d'aromatisation des hydrocarbures contenant moins de 9 atomes de carbone par molécule en présence de zéolithes a déjà fait l'objet de brevets et de publications. Plusieurs systèmes catalytiques à base de zéolithe MFI sont revendiqués, ces systèmes pouvant se distinguer par les ajouts qu'ils contiennent. Schématiquement, on peut distinguer :

(i) les systèmes contenant du gallium, et,

(ii) les systèmes contenant du zinc.

Ces systèmes souffrent tous d'un défaut important, à savoir une sélectivité élevée en méthane. Pour améliorer les performances de ces systèmes catalytiques plusieurs solutions ont été proposées dont l'ajout de platine (Z. Jin, Y. Makino, A. Miyamoto, T. Inui, Chem. Express, 2, p.515, 1987). L'utilisation d'une zéolithe MFI non acide dopée avec divers éléments métalliques a également été revendiquée (Y Chen et al., WO 8904818).

Récemment (demande de brevet français 90/10451 ), il a été découvert par la demanderesse que l'utilisation de catalyseurs composites contenant une zéolithe MFI d'une part, et d'autre part un support ou une matrice généralement amorphe sur laquelle est déposé un métal noble de la famille du platine et au moins un métal additionnel comme l'étain, le plomb ou l'indium, ledit support contenant également éventuellement au moins un métal alcalin ou alcalino-terreux (tels le lithium ou le potassium), conduit à des performances catalytiques dans les réactions d'aromatisation des paraffines de 2 à 9 atomes de carbone nettement améliorées par rapport aux systèmes de l'art antérieur.

L'utilisation de tels catalyseurs permet en particulier de limiter les réactions qui conduisent à la formation de méthane, produit non désiré.

Les travaux de recherche effectuées par la demanderesse l'ont conduite à découvrir que, de façon surprenante, l'utilisation d'un catalyseur composite contenant une zéolithe MFI sous forme hydrogène, du gallium sous forme oxyde et une matrice généralement amorphe sur laquelle est déposé au moins un métal noble de la famille du platine (palladium, platine, nickel), au moins un métal additionnel choisi dans le groupe constitué par l'étain, le germanium, le plomb, l'indium, le plomb, le gallium et le thallium, les métaux du groupe VIII tels que le cuivre, l'or, l'argent, le nickel, le ruthénium, le fer et les métaux du groupe VI tels que le chrome, le molybdène, le tungstène, ladite matrice contenant éventuellement au moins un alcalin ou un alcalino-terreux (de préférence le lithium ou le potassium), conduit à des performances catalytiques dans les réactions d'aromatisation des paraffines contenant de 2 à 9 atomes de carbone par molécule nettement améliorées par rapport aux catalyseurs de l'art antérieur.

La zéolithe MFI contenue dans le catalyseur de la présente invention peut être préparée par toutes les techniques décrites dans l'art antérieur. Ainsi la synthèse de ladite zéolithe peut être réalisée en milieu classique OH⁻ en présence ou en absence d'agent organique et/ou d'alcool. Le document "Synthesis of high silica zeolites, P. Jacobs and J. Martens, Studies in Surface Science and Catalysis, Vol. 33, 1987" décrit la synthèse classique de la zéolithe MFI. La zéolithe MFI utilisée dans la présente invention peut également avoir été synthétisée dans des milieux moins classiques comme par exemple le milieu fluorure en présence (brevet EP-A-172068) ou en absence (demande de brevet français 90/16529) de composé organique. La zéolithe utilisée dans la présente invention contient, dans sa charpente cristallisée, du silicium et au moins un élément choisi dans le groupe formé par l'aluminium et le gallium.

2

Après l'étape de synthèse, la zéolithe MFI est transformée en une forme hydrogène, notée H-MFI, par élimination pratiquement totale des composés organiques et/ou des cations alcalins ou alcalino-terreux qu'elle contient éventuellement après synthèse. Toutes les techniques décrites dans l'art antérieur peuvent être utilisées pour le passage à la forme hydrogène, comme par exemple les échanges ioniques suivis ou non de calcination ou les traitements chimiques divers.

Toutes les zéolithes synthétisées dans l'un des systèmes suivants : Si-Al, Si-Al-Ga, Si-Ga, conviennent pour la présente invention. Cependant, leur rapport Si/T où T représente Al et/ou Ga, est généralement supérieur à 7, de préférence supérieur à 25 et de manière encore plus préférée compris entre 40 et 500.

La zéolithe H-MFI, utilisée dans la présente invention, peut être soit soumise telle quelle à un dépôt de gallium soit mélangée avec les autres constituants du catalyseur, le gallium étant alors introduit ultérieurement dans ledit mélange.

De nombreuses techniques de dépôt de gallium peuvent être utilisées dans la présente invention parmi lesquelles on peut citer :
- les échanges ioniques grâce à l'utilisation de sels de gallium en solution aqueuse, par exemple du nitrate de gallium, du chlorure de gallium, de l'hydroxyde de gallium,
- les imprégnations par des solutions desdits sels de gallium.

La teneur en gallium déposé sur le catalyseur composite est comprise entre 0,01 et 10 % en poids, de préférence entre 0,03 et 4 % en poids.

La matrice comprend au moins un oxyde réfractaire, et en particulier au moins un oxyde d'un métal choisi dans le groupe constitué par le magnésium, l'aluminium, le titane, le zirconium, le thorium, le silicium et le bore. De plus, elle peut aussi comprendre du charbon.

La matrice préférée est l'alumine, dont la surface spécifique peut être avantageusement comprise entre 10 et 600 m$^2$/g et de préférence entre 150 et 400 m$^2$/g.

Le catalyseur composite de la présente invention peut être préparé suivant deux voies dont le principe est donné ci-après, la réalisation pratique étant connue de l'homme de l'art.

Première voie

La zéolithe H-MFI est mélangée avec la matrice. Ce mélange peut être réalisé entre deux poudres, entre deux solides mis préalablement en forme, entre une poudre et un solide mis préalablement en forme. On peut également mettre en forme conjointement les deux solides par toutes les techniques décrites dans l'art antérieur : pastillage, extrusion, dragéification, coagulation en goutte, séchage par atomisation. Lors de ces opérations de mise en forme, on peut si nécessaire ajouter un additif de mise en forme, choisi dans le groupe constitué par la silice et l'alumine. Ainsi on a procédé au mélange de la zéolithe avec la matrice et à la mise en forme. Après mélange et mise en forme, on procède au dépôt des métaux et du gallium sur l'ensemble constitué de la matrice et de la zéolithe, l'ordre de dépôt étant peu important. On considère alors que la majeure partie des métaux, c'est-à-dire de 30 à 100 % en poids et de préférence de 60 à 100 % en poids par rapport au catalyseur composite, se retrouve sur la matrice.

Deuxième voie

On dépose préalablement les métaux sur la matrice d'une part, et le gallium sur la zéolithe H-MFI d'autre part. Puis on procède au mélange de la zéolithe H-MFI contenant du gallium avec la matrice contenant des métaux et à leur mise en forme, à la mise en forme étant obtenue dans les mêmes conditions que précédemment. Dans une variante, la zéolithe, sur laquelle a été déposé le gallium, peut être mélangée à la matrice à l'une quelconque des étapes de dépôt des métaux sur la matrice.

La méthode préférée de préparation consiste à déposer le gallium sur la zéolithe, à déposer les métaux sur la matrice, puis ensuite à introduire la zéolithe chargée en gallium dans la matrice chargée en métaux par mise en forme des deux poudres. La mise en forme est de préférence effectuée après un broyage micronique, qui peut être réalisé en utilisant la technique du broyage humide.

Le catalyseur composite contient entre 1 et 99 % en poids de zéolithe, le complément à 100 % étant constitué par la matrice, les métaux et le gallium. La proportion respective de zéolithe et de matrice varie dans une large gamme, car elle dépend d'une part du rapport Si/T de la zéolithe, où T est Al et/ou Ga, et d'autre part de la teneur en métaux de la matrice dans le cas de la méthode préférée de préparation.

La matrice contenant les métaux, dans le cas de la méthode préférée de préparation, est généralement préparée suivant les procédures décrites dans la demande de brevet français 90/10451, dont une partie est reproduite dans ce qui suit.

On utilise pour l'imprégnation des métaux, soit une solution commune des métaux que l'on désire introduire, soit des solutions distinctes pour le métal noble de la famille du platine et pour le métal additionnel et éventuellement l'élément choisi dans le groupe constitué par les métaux alcalins et les alcalino-terreux. Quand on utilise plusieurs solutions, on peut procéder à des séchages et/ou à des calcinations intermédiaires. On termine habituellement par une calcination, par exemple entre 500 et 1000 °C, de préférence en présence d'oxygène moléculaire, par exemple en effectuant un balayage d'air.

Le métal noble de la famille du platine peut être incorporé dans la matrice par imprégnation de ladite matrice à l'aide d'une solution, aqueuse ou non, contenant un sel ou un composé du métal noble. Le platine est généralement introduit dans la matrice sous forme d'acide chloroplatinique, mais pour tout métal noble peuvent être également utilisés des composés ammoniaqués ou des composés tels que par exemple le chloroplatinate d'ammonium, le dichlorure de platine dicarbonyle, l'acide hexahydroxyplatinique, le chlorure de palladium, le nitrate de palladium.

Le métal additionnel choisi dans le groupe constitué par l'étain, le germanium, le plomb, l'indium, le plomb, le gallium et le thallium, les métaux du groupe VIII tels que le cuivre, l'or, l'argent, le nickel, le ruthénium, le fer et les métaux du groupe VI tels que le chrome, le molybdène, le tungstène peut être introduit par l'intermédiaire de composés tels que par exemple les chlorures, les bromures et les nitrates d'étain, les halogénures, le nitrate, l'acétate et le carbonate de plomb, le chlorure et l'oxalate de germanium, le nitrate et le chlorure d'indium.

L'élément choisi dans le groupe constitué par les alcalins et les alcalino-terreux, de préférence le lithium ou le potassium, peut être introduit par l'intermédiaire de composés tels que par exemple l'halogénure, le nitrate, le carbonate, le cyanure et l'oxalate dudit élément.

Un procédé de préparation, que nous détaillons ci-après, comprend par exemple les étapes suivantes :
a) Introduction sur la matrice d'au moins un élément choisi dans le groupe constitué par les alcalins et les alcalino-terreux.
b) Calcination du produit obtenu à l'étape a).
c) Introduction sur la matrice d'au moins un métal noble de la famille du platine.
d) Calcination du produit obtenu à l'étape c).
e) Introduction sur le produit obtenu à l'étape d) d'au moins un métal additionnel M.

Si on n'utilise pas de métal alcalin ou alcalino-terreux, on effectue uniquement les étapes c), d) et e) dudit procédé de préparation.

L'emploi dans la présente invention d'au moins un métal noble de la famille du platine peut à titre d'exemple se faire grâce à l'utilisation de composés ammoniaqués.

Dans le cas du platine, on peut citer par exemple les sels de platine IV hexamines de formule $Pt(NH_3)_6X_4$; les sels de platine IV halogénopentamines de formule $(PtX(NH_3)_5)X_3$; les sels de platine N tétrahalogénodiamines de formule $PtX_4(NH_3)_2$ ; les complexes de platine avec les halogènes-polycétones et les composés halogénés de formule $H(Pt(aca)_2X)$; X étant un halogène choisi dans le groupe formé par le chlore, le fluor, le brome et l'iode , et de préférence X étant le chlore, et aca représentant le reste de formule $C_5H_7O_2$ dérivé de l'acétylacétone.

L'introduction du métal noble de la famille du platine est de préférence effectuée par imprégnation à l'aide d'une solution aqueuse ou organique de l'un des composés organométalliques cités ci-dessus. Parmi les solvants organiques utilisables, on peut citer les hydrocarbures paraffiniques, naphténiques ou aromatiques, et les composés organiques halogénés ayant par exemple de 1 à 12 atomes de carbone par molécule. On peut citer par exemple le n-heptane, le méthylcyclohexane, le toluène et le chloroforme. On peut aussi utiliser les mélanges de solvants.

Après introduction du métal noble de la famille du platine, le produit obtenu est éventuellement séché puis il est calciné de préférence à une température comprise entre 400 et 1000 °C.

Après cette calcination, on procède à l'introduction d'au moins un métal additionnel, précédée éventuellement d'une réduction à l'hydrogène à haute température, par exemple entre 300 et 500 °C. Le métal additionnel M peut être introduit sous la forme d'au moins un composé organique choisi dans le groupe constitué par les complexes dudit métal, en particulier les complexes polycétoniques du métal M et les hydrocarbylmétaux tels que les alkyles, les cycloalkyles, les aryles, les alkylaryles et les arylalkyles métaux.

L'introduction du métal M est avantageusement effectuée à l'aide d'une solution du composé organométallique dudit métal dans un solvant organique. On peut également employer des composés organohalogénés du métal M. Comme composés du métal M, on peut citer en particulier le tétrabutylétain dans le cas où M est l'étain, le tétraéthylplomb dans le cas où M est le plomb et le triphénylindium dans le cas où M est l'indium.

Le solvant d'imprégnation est choisi dans le groupe constitué par les hydrocarbures paraffiniques, naphténiques ou aromatiques contenant de 6 à 12 atomes de carbone par molécule et les composés organiques halogénés contenant de 1 à 12 atomes de carbone par molécule. On peut citer par exemple le n-heptane, le méthylcyclohexane et le chloroforme. On peut aussi utiliser des mélanges des solvants définis ci-dessus.

Dans le cas où l'on n'utilise pas le procédé de préparation tel que décrit ci-dessus, on peut aussi envisager d'introduire au moins un métal additionnel M avant l'introduction d'au moins un métal noble de la famille de platine. Si le métal M est introduit avant le métal noble, le composé du métal M utilisé est généralement choisi dans le groupe constitué par l'halogénure, le nitrate, l'acétate, le tartrate, le carbonate et l'oxalate du métal M. L'introduction est alors avantageusement effectuée en solution aqueuse. Dans ce cas, avant de procéder à l'introduction d'au moins un métal noble, on procède à une calcination sous air à une température comprise entre 400 et 1000 °C.

Le catalyseur composite contient :

1) en poids par rapport à la matrice:
   – de 0,01 à 2 % et de préférence de 0,1 à 0,5 % d'au moins un métal noble de la famille du platine,
   – de 0,005 à 2 %, de préférence de 0,01 à 0,5 %, d'étain dans le cas où le catalyseur contient de l'étain, de 0,005 à 0,7 %, de préférence de 0,01 à 0,6 %, d'au moins un métal choisi dans le groupe constitué par le germanium, l'indium, le plomb, le gallium et le thallium, les métaux du groupe VIII tels que le cuivre, l'or, l'argent, le nickel, le ruthénium, le fer et les métaux du groupe VI tels que le chrome, le molybdène, le tungstène dans le cas où le catalyseur contient au moins un métal additionnel dudit groupe, la teneur globale en métaux choisis dans l'ensemble constitué par l'étain et ledit groupe étant comprise entre 0,02 et 1,20 %, de préférence entre 0,02 et 1,0 %, et de manière encore plus préférée entre 0,03 et 0,80 %.
   – éventuellement de 0,01 à 4 %, de préférence de 0,1 à 0,6 % d'au moins un métal choisi dans le groupe constitué par les alcalins et les alcalino-terreux, de préférence choisi dans le groupe constitué par le lithium et le potassium.

2) entre 1 et 99% en poids de zéolithe MFI forme hydrogène,

3) entre 0,01 et 10% en poids, de préférence entre 0,03 et 4%, de gallium.

Dans le procédé selon l'invention, à l'issue de la préparation, le catalyseur mis en forme contient une zéolithe H-MFI, du gallium, des métaux et une matrice, et on procède à une calcination sous air à une température comprise entre 450 et 1000 °C. Le catalyseur ainsi calciné peut avantageusement subir un traitement d'activation sous hydrogène à haute température, par exemple comprise entre 300 et 500 °C. La procédure de traitement sous hydrogène consiste par exemple en une montée lente de la température sous courant d'hydrogène jusqu'à la température maximale de réduction, comprise généralement entre 300 et 500 °C et de préférence entre 350 et 450 °C, suivie d'un maintien à cette température pour une durée comprise en général entre 1 et 6 heures.

Le catalyseur de la présente invention décrit précédemment est mis en oeuvre pour l'aromatisation des alcanes contenant de 2 à g atomes de carbone par molécule, en présence ou non d'oléfnes. Cette réaction revêt un intérêt particulier car elle peut permettre, par exemple, de valoriser des résidus d'opérations de raffinage en les transformant en des produits à plus haute valeur ajoutée (benzène, toluène et xylènes) tout en contribuant à la production de quantités importantes d'hydrogène indispensable pour les procédés d'hydrotraitement par exemple.

La charge qui contient des composés contenant de 2 à 9 atomes de carbone par molécule est mise en contact avec le catalyseur de la présente invention à une température comprise entre 400 et 700 °C.

Les exemples qui suivent précisent l'invention sans toutefois en limiter la portée.

Exemple 1 : préparation de l'alumine renfermant du platine, de l'étain et du lithium (catalyseur A).

L'alumine utilisée a une surface spécifque de 240 m²/g et un volume poreux de 0,48 cm³/g.

On ajoute à 100 g de support d'alumine 100 cm³ d'une solution aqueuse de nitrate de lithium.

On laisse en contact 6 heures, on essore, on sèche 1 heure à 100-120°C puis on calcine 2 heures à 530°C.

Sur le produit calciné contenant le lithium, on procède à l'imprégnation de l'étain: une solution aqueuse d'acétate d'étain est mise en contact avec le support d'alumine à raison de 100 cm³ de solution pour 100 g de support pendant 6 heures. Le solide obtenu est alors essoré et séché 1 heure à 100-120°C puis calciné à 530°C.

On procède alors sur le solide calciné contenant le lithium et l'étain à l'imprégnation du platine, en ajoutant au solide 100 cm³ d'une solution d'acétylacétonate de platine dans le toluène. La concentration en platine de cette solution est égale à 3 g/l. On laisse 6 heures en contact, on sèche 1 heure à 100-120°C puis on calcine 2 heures à 530°C. Puis on réduit sous courant d'hydrogène sec pendant 2 heures à 450°C.

L'alumine contient alors, en poids, 0,30 % de platine, 0,3 % d'étain et 0,5 % de lithium.

Exemple 2: préparation de l'alumine renfermant du platine, de l'étain et du lithium (catalyseur B).

On ajoute à 100 g de support d'alumine, 100 cm³ d'une solution aqueuse de nitrate de lithium, on laisse en contact 6 heures, on essore, on sèche en 1 heure à 100-120°C puis on calcine sous courant d'air sec pendant 2 heures à 530°C.

Sur le produit calciné contenant le lithium, on procède à l'imprégnation du platine de la même façon que le produit A.

Après réduction, le produit contenant le lithium et le platine est immergé dans le n-heptane à raison de 100 g de solide pour 300 cm³ de solvant hydrocarboné. Ensuite, on injecte dans le n-heptane contenant le catalyseur, 3 g d'une solution de tétra-n-butylétain dans le n-heptane (solution à 10 % poids d'étain). Le contact entre le solide contenant le platine et la solution de tétra-n-butylétain est maintenu pendant 6 heures à la température de reflux de l'heptane. La solution d'imprégnation est alors évacuée et on procède à 3 lavages par du n-heptane pur à la température de reflux du n-heptane. Le catalyseur est ensuite séché. Il subit alors une calcination sous air pendant 2 heures à 500°C suivie d'une réduction sous courant d'hydrogène à 450°C avant d'être chargé dans le réacteur.

L'alumine renferme alors, en poids, 0,3 % de platine, 0,3 % d'étain et 0,5 % de lithium.

Exemple 3 : zéolithe MFI forme hydrogène et catalyseur C contenant cette zéolithe H-MFI et du gallium.

On utilise une zéolithe H-MFI forme hydrogène fournie par la société CONTEKA sous la référence CBV 1502. Cette zéolithe H-MFI est caractérisée par un rapport Si/Al égal à 75, une teneur en sodium égale à 0,016 % en poids et un volume poreux mesuré par adsorption d'azote à 77K de 0, 174 cm³/g.

Le gallium est déposé sur cette zéolithe par échange ionique. La solution d'échange est préparée à partir de nitrate de gallium $Ga(NO_3)_3$ de normalité 0,15 N. Le pH de la solution de nitrate de gallium est ajusté à la valeur de 2 par ajout d'ammoniaque.

La teneur en gallium atteinte, après trois échanges ioniques successifs de la zéolithe H-MFI avec la solution décrite ci-dessus, dans le catalyseur C ainsi obtenu est égale à 3,3 % en poids.

Exemple 4

On se propose de transformer une charge constituée d'un mélange d'hydrocarbures contenant de 5 à 6 atomes de carbone par molécule. On opère pour cela en présence d'un des catalyseurs suivants: le catalyseur constitué par la zéolithe H-MFI décrite dans l'exemple 3 seul ou mélangé au catalyseur A de l'exemple 1 ou mélangée au catalyseur B de l'exemple 2, et le catalyseur C de l'exemple 3, seul ou mélangé au catalyseur A de l'exemple 1 ou mélangé au catalyseur B de l'exemple 2. Les mélanges sont tous des mélanges équi-massiques et ils sont utilisés après pastillage.

Ces catalyseurs ont été testés en transformation d'une charge $C_5$-$C_6$ dont la composition est la suivante (exprimée en % poids) :

Paraffines    $C_5$..........90%
              $C_6$..........5,4 %
Naphtènes    $C_5$..........3,7 %
              $C_6$..........0,9 %

Les conditions opératoires sont les suivantes :
– température....480°C
– pression....2,5 bars
– pph....3 h⁻¹

Les résultats du test sont reportés dans le tableau 1.

Tableau 1

| Catalyseur | conversion (%poids) | Sélectivités (%poids) | | | | |
|---|---|---|---|---|---|---|
| | | CH$_4$ | C$_2$H$_6$+C$_2$H$_4$ | C$_3$H$_8$+C$_3$H$_6$ | C$_4$H$_{10}$ | Aromatiques |
| zéolithe MFI (comparatif) | 92 | 30 | 25 | 15 | 20 | 10 |
| catalyseur C (comparatif) | 93 | 7 | 18 | 29 | 8 | 38 |
| mélange : 50% cata. A 50% zéolithe H-MFI | 90 | 6 | 12 | 14 | 8 | 60 |
| mélange : 50% cata. B 50% zéolithe H-MFI | 92 | 5 | 11 | 15 | 7 | 62 |
| mélange : 50% cata. A 50% cata. C | 91 | 6 | 9 | 15 | 7 | 63 |
| mélange : 50% cata. B 50% cata. C | 93 | 5 | 9 | 14 | 8 | 64 |

On constate donc que les mélanges catalyseur A - catalyseur C et catalyseur B - catalyseur C selon l'invention conduisent à des sélectivités en produits aromatiques supérieures.

**Revendications**

**1 -** Catalyseur composite contenant :
– de 1 à 99% en poids d'une zéolithe de structure MFI sous forme hydrogène, contenant dans sa charpente le silicium et au moins un élément choisi dans le groupe formé par l'aluminium et le gallium,
– une matrice
– du gallium
– au moins un métal noble de la famille du platine, au moins un métal additionnel choisi dans le groupe constitué par l'étain, le germanium, l'indium, le plomb, le gallium et le thallium, ou bien parmi les métaux du groupe VIII tels que le cuivre, l'or, l'argent, le nickel, le ruthénium, le fer ou encore parmi les métaux du groupe VI tels que le chrome, le molybdène ou le tungstène

**2.** Catalyseur composite selon la revendication 1 et contenant au moins un composé choisi dans le groupe constitué par les métaux alcalins et les alcalino-terreux,

**3 -** Catalyseur composite selon l'une des revendications 1 à 2 dans lequel la teneur en gallium est comprise entre 0,01 et 10 % en poids.

**4 -** Catalyseur composite selon l'une des revendications 1 à 3 dans lequel ledit catalyseur contient, en poids par rapport à la matrice :
– de 0,01 à 2 % d'au moins un métal noble de la famille du platine,
– de 0,005 à 2 % d'étain dans le cas où le catalyseur contient de l'étain, ou de 0,005 à 0,7 % d'au moins un métal choisi dans le groupe constitué par le germanium, l'indium, le plomb, le gallium et le thallium, et par les métaux du groupe VIII tels que le cuivre, l'or, l'argent, le nickel, le ruthénium, le fer et par les métaux du groupe VI tels que le chrome, le molybdène, le tungstène dans le cas où le catalyseur contient au moins un métal dudit groupe, la teneur globale en métaux choisis dans l'ensemble constitué par l'étain et ledit groupe étant comprise entre 0,02 et 1,20 %.

5. Catalyseur composite selon la revendication 4 et qui contient, en poids par rapport à la matrice :
– de 0,01 à 4 % d'au moins un métal choisi dans le groupe constitué par les alcalins et les alcalino-terreux.

6 - Catalyseur composite selon l'une des revendications 1 à 5 dans lequel la matrice est de l'alumine.

7 - Préparation du catalyseur composite selon l'une des revendications 1 à 6 dans lequel on procède au mélange de la zéolithe avec la matrice et à la mise en forme, puis on procède au dépôt des métaux et du gallium; enfin on procède à une calcination.

8 - Préparation du catalyseur composite selon l'une des revendications 1 à 6 dans lequel on dépose préalablement les métaux sur la matrice et le gallium sur la zéolithe; puis l'on procède au mélange de la zéolithe MFI avec la matrice et à la mise en forme, enfin on procède à une calcination.

9 - Préparation du catalyseur composite selon l'une des revendications 7 et 8 dans laquelle, à l'issue de la calcination, on procède à une activation sous hydrogène à haute température.

10 - Utilisation du catalyseur composite défini selon l'une des revendications 1 à 6 ou préparé selon l'une des revendications 7 à 9 pour l'aromatisation d'hydrocarbures contenant de 2 à 9 atomes de carbone par molécule.

EP 0 507 656 A1

**Office européen des brevets**

# RAPPORT DE RECHERCHE EUROPEENNE

Numero de la demande

EP    92 40 0844

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| Y | EP-A-0 244 162 (THE BRITISH PETROLEUM)<br>* revendications 1-10 *<br>--- | 1-10 | B01J29/04<br>B01J29/32<br>C07C2/00 |
| P,Y | EP-A-0 471 599 (INSTITUT FRANCAIS DU PETROLE)<br>* revendications 1-6 *<br>* page 2, ligne 56 - page 3, ligne 11 *<br>--- | 1-10 | |
| P,A | EP-A-0 472 462 (INSTITUT FRANCAIS DU PETROLE)<br>--- | | |
| A | US-A-4 886 927 (J. W. SACHTLER)<br>--- | | |
| A | EP-A-0 351 312 (INSTITUT FRANCAIS DU PETROLE)<br>--- | | |
| A | US-A-4 806 701 (V. K. SHUM)<br>--- | | |
| A | EP-A-0 162 590 (THE BRITISH PETROLEUM COMPANY)<br><br>----- | | |

DOMAINES TECHNIQUES RECHERCHES (Int. Cl.5)

B01J
C07C

Le présent rapport a été établi pour toutes les revendications

| Lieu de la recherche | Date d'achèvement de la recherche | Examinateur |
|---|---|---|
| LA HAYE | 16 JUILLET 1992 | THION M.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)

9